# FASCICULE DE BREVET EUROPEEN

(11) **EP 0 923 367 B1**
(45) Date de publication et mention de la délivrance du brevet: **19.12.2001**
(21) Numéro de dépôt: 96934945.5
(22) Date de dépôt: 21.10.1996
(51) Int. Cl.: A61K 7/48

(54) **UTILISATION EN COSMETIQUE D'UN GEL RIGIDE ET COMPOSITIONS COSMETIQUES OU DERMATOLOGIQUES MISES EN OEUVRE**
VERWENDUNG FÜR KOSMETISCHE ZWECKE EINES STEIFEN GELS, UND ANGEWANDTE KOSMETISCHE UND DERMATOLOGISCHE ZUSAMMENSETZUNGEN
COSMETIC USE OF A RIGID GEL, AND COSMETIC OR DERMATOLOGICAL COMPOSITIONS THEREFOR

(30) Priorité: 06.11.1995 FR 9513095
(43) Date de publication de la demande: 23.06.1999
(73) Titulaire: L'OREAL, 75008 Paris (FR)
(72) Inventeur: ROULIER, Véronique, F-75010 Paris (FR); MELLUL, Myriam, F-94240 L'Hay-les-Roses (FR); DAUBIGE, Thérèse, F-77480 Mousseaux les Bray (FR); HOLZ, Katrin, CH-1009 Pully (CH)
(74) Mandataire: Dodin, Catherine
(86) Numéro de dépôt international: FR9601642
(87) Numéro de publication internationale: WO9717055

(56) Documents cités:
- EP-A- 0 412 865
- EP-A- 0 651 991
- DE-A- 4 320 401
- US-A- 5 034 216
- DATABASE WPI Section Ch, Week 8525 Derwent Publications Ltd., London, GB; Class A96, AN 85-149123 XP002011387 & JP 60 081 120 A (SHISEIDO KK) , 9 Mai 1985

## Description

La présente invention concerne l'utilisation comme produits cosmétiques de gels rigides susceptibles d'être obtenus par extrusion ainsi que les compositions cosmétiques et dermatologiques mises en oeuvre.

On connaît dans l'industrie cosmétique diverses formes de produits sous forme solide notamment dans le domaine du maquillage comme les bâtons ou "sticks" de rouge à lèvres, de fond de teint ou d'ombre à paupières ; dans le domaine du soin de la peau ou des lèvres tels que les crayons réparateurs des lèvres, les bâtons ou "sticks" dépigmentants, démaquillants ou hydratants ; dans le domaine de l'hygiène comme les sticks déodorants, les sticks ou les pains moussants pour le rasage ou pour le lavage de la peau.
On connaît notamment, par le document EP412865, un gel aqueux contenant en suspension des sphéroïdes d'une substance lipidique solide non hydrophile fondant par application sur le corps.

La demanderesse a découvert de manière surprenante de nouvelles compositions à usage cosmétique ou dermatologique sous la forme d'un gel rigide susceptible d'être obtenu par un procédé d'extrusion.

On entendra par gel rigide, dans toute la description, tout gel présentant une résistance à la compression supérieure ou égale à 50 grammes, à température ambiante, après pénétration par une sonde cylindrique de révolution ayant un diamètre de 0,8 cm dans la matrice du gel dans une épaisseur de 5 mm, à une vitesse de 1 mm/s, maintien de ladite sonde dans la matrice du gel pendant 15 secondes et retrait de ladite sonde de la matrice du gel à une vitesse de 1mm/s ; la résistance à la compression étant mesurée avec un analyseur de texture du type TAXT2 commercialisé par la société RHEO.

Les compositions selon l'invention peuvent se présenter sous forme de bâton, de crayon ou de pain et constituer en elles-mêmes de nouveaux types de produits de maquillage tels que des rouges à lèvres, des fonds de teint, des ombres à paupières ; de nouveaux types de produits de soin et/ou de conditionnement des cheveux tels que des gels durs de coiffage ; des nouveaux types de produits sous forme de bâton pour le soin ou bien pour l'hygiène du visage ou du corps.

Les compositions selon l'invention peuvent être aqueuses et présentent une matrice constituée d'un réseau gélifié aqueux. Elles peuvent également se présenter sous forme anhydre avec un matrice constituée d'un réseau gélifié déshydraté.

Les compositions aqueuses selon l'invention peuvent être partiellement réhydratables en surface en contact avec de l'eau au moment de l'utilisation, permettre ainsi un bon reiarguage des produits cosmétiquement actifs sur la matière kératinique à traiter, et reprendre rapidement, après séchage, leur forme solide initiale sans altération. Elles peuvent alors être réutilisées ultérieurement par simple hydratation partielle en surface. A l'état de repos, elles sont stables à la conservation et ne sont pas collantes au toucher.

Les compositions aqueuses de l'invention peuvent en particulier constituer des compositions en forme de bâton pour le maquillage apportant directement sur la peau, de manière inattendue, l'effet couleur, la sensation de fraîcheur et le glissant sans qu'il soit nécessaire d'incorporer de fortes quantités de corps gras. En outre, elles ne produisent pas à l'application d'effet gras et collant au toucher ni de phénomène de migration dans les ridules, sur le visage ou le bord des paupières, contrairement aux bâtons de maquillage classiques contenant de fortes concentrations de corps gras (huiles ou cires) en vue d'obtenir l'effet couleur et le glissant. Elles peuvent, en outre, contenir des actifs cosmétiques ou dermatologiques hydrosolubles qui ne peuvent être incorporés dans les bâtons de maquillage courants, généralement anhydres

Les compositions selon l'invention peuvent contenir suffisamment de charges minérales et/ou de charges organiques pour obtenir un bon délitage et des qualités de douceur satisfaisantes.

L'obtention de bâtons ou «sticks» dont la matrice est constituée d'un réseau gélifié aqueux, ayant une rigidité suffisamment élevée, nécessite la présence d' au moins un agent gélifiant hydrosoluble à des concentrations élevées, en général supérieures à 20% en poids. Jusqu'à présent, il n'était pas possible de fabriquer de tels gels aqueux solides selon les techniques classiques tels que la fabrication par coulée.

La demanderesse a découvert de façon inattendue que l'on pouvait réaliser des sticks rigides, homogènes et stables, dont la matrice gélifiée pouvait contenir plus de 20% en poids d'agent gélifiant hydrosoluble et de fortes quantités de charges, selon un procédé d'extrusion que l'on définira plus loin.

Les compositions selon l'invention notamment les compositions anhydres ayant une matrice constituée d'un réseau gélifié déshydraté, permettent de réaliser des poudres de maquillage pouvant contenir, dans des quantités importantes, des cires conférant de bonnes propriétés de tenue de film, de glissant et de mat. Ce fait est d'autant plus surprenant que les poudres compactées de maquillage classiques ne peuvent pas, en général, contenir des quantités importantes de corps gras tels que des cires (plus de 10% en poids). Leur incorporation dans des poudres conduit à des produits qui cirent et qui ne peuvent être délités.

Elles peuvent également contenir des gommes de silicone qui sont généralement difficiles à incorporer de façon homogène à la fois dans les phases anhydres et dans les phases aqueuses.

Les compositions selon l'invention, notamment les compositions anhydres ayant une matrice constituée d'un réseau gélifié déshydraté, peuvent de plus contenir des quantités importantes de charges difficilement compactables conférant un toucher très doux et non-gras. Par charge difficilement compactable, on entend une matière première qui, à partir d'un certain pourcentage qui dépendra de la matière en question, ne peut être compactée au moyen d'une presse mécanique. Ces types de charge ne peuvent pas être utilisées à des concentrations importantes dans les produits de maquillage sous forme de poudre compactée. De plus, les produits de maquillage les contenant, même en faible quanité, ne présentent pas une bonne intégrité au stockage, une bonne solidité aux choc et/ou une surface plane convenable.

Les compositions de l'invention notamment les compositions anhydres ayant une matrice constituée d'un réseau gélifié déshydraté, peuvent être utilisées sous forme de "stick" poudreux compact comme une poudre de maquillage classique sans présenter les inconvénients évoqués ci-dessus. Ces compositions anhydres peuvent également être réduites en poudre et utilisées de façon classique comme poudre de maquillage sans présenter les inconvénients évoqués ci-dessus.

Les compositions selon l'invention sont des gels rigides contenant dans un milieu cosmétiquement acceptable au moins 20% en poids d'au moins un agent gélifiant hydrosoluble ou hydrophile et au moins une substance cosmétique ou dermatologique. Ces gels rigides sont susceptibles d'être obtenus à partir dudit agent gélifiant en présence d'eau, par mélange, malaxage, compression et extrusion dans un extrudeur bi-vis.

Ces gels rigides sont soit sous forme aqueuse et présentent une matrice constituée d'un réseau gélifié aqueux ou bien sous forme anhydre avec une matrice constituée d'un réseau gélifié déshydraté et susceptibles d'être obtenus par déshydratation du réseau gélifié par un procédé de séchage classique.

Les gels rigides selon l'invention présentent en général une résistance à la compression supérieure ou égale à 50 grammes, à température ambiante, après pénétration par une sonde cylindrique de révolution ayant un diamètre de 0,8 cm dans la matrice du gel dans une épaisseur de 5 mm, à une vitesse de 1 mm/s, maintien de ladite sonde dans la matrice du gel pendant 15 secondes et retrait de ladite sonde de la matrice du gel à une vitesse de 1 mm/s ; la résistance à la compression étant mesurée avec un analyseur de texture du type TAXT2 commercialisé par la société RHEO.

De façon préférentielle, pour les gels rigides conformes à l'invention, on observe une courbe relative à la rigidité du gel et au dépôt du gel sur la sonde en fonction du temps, dans les conditions telles que définies ci-dessus, présentant un pic positif correspondant à la force de compression du gel après pénétration de la sonde mais ne présentant pas de pic négatif correspondant à un dépôt de gel sur la sonde après retrait de celle-ci.

Les compositions selon l'invention contiennent un ou plusieurs agents gélifiants hydrosolubles ou hydrophiles.

Les agents gélifiants hydrosolubles ou hydrophiles présents dans les compositions de l'invention, sont choisis de préférence dans le groupe formé par :
- les extraits d'algue tels que l'agar-agar, les carraghénanes, les alginates ;
- tes extraits de graines tels que la gomme de caroube, la gomme de guar ;
- les exudats de plantes tels que la gomme arabique, la gomme karaya, la gomme adragante, la gomme de gatty ;
- les exudats de microorganismes tels que la gomme de xanthane, la cellulose ou ses dérivés comme la carboxyméthylcellulose, l'hydroxypropylcellulose, la méthylcellulose, l'hydroxypropylméthylcellulose ou l'hydroxyéthylcellulose ainsi que les celluloses modifiées notamment par greffage de groupement alkyle ;
- les extraits de fruits tels que les pectines ;
- les agents gélifiants d'origine animale tels que la gélatine, les caséïnates ;
- les polymères synthétiques gélifiants hydrosolubles tels que les acides polyacryliques réticulés tels que les "Carbopol" ou "Pemulen" de la Société Goodrich ;
- les dérivés du silicium tels que les hectorites synthétiques comme les produits "Laponite RD et RDS" vendus par la société WAVERLY, les silicates d'aluminium et de magnésium comme le produit "Veegum" vendu par la société VANDERBILT.

Les agents gélifiants hydrosolubles ou hydrophiles sont présents dans les compositions selon l'invention, préférentiellement, dans des concentrations allant de 20 à 80% en poids par rapport au poids total de la composition.

Les compositions selon l'invention peuvent contenir en plus des charges minérales et/ou des charges organiques.

Les charges utilisées dans les compositions de l'invention sont des particules cosmétiques ou dermatologiques insolubles dans le milieu formé par la matrice amidonnée.

Elles sont présentes dans les compositions de l'invention, à des concentrations allant jusqu'à 80% en poids par rapport au poids total de la composition, selon l'application choisie.

Lorsque les charges ont une densité très faible, notamment inférieure à 0,1 g.cm³,elles sont de préférence présentes dans des concentrations allant jusqu'à 40% en poids par rapport à la composition finale.
Lorsque les charges ont une densité plus élevée, notamment supérieure à 0,5 g.cm⁻³, elles sont de préférence présentes à raison de 2-80% en poids par rapport à la composition finale.

Les charges utilisées selon l'invention sont choisies de préférence parmi les charges minérales ou organiques, de structure lamellaire ou sphérique ou leurs mélanges. Elles peuvent être compactables ou difficilement compactables.

Chaque type de charges permet d'apporter des qualités particulières et différentes à la composition selon l'invention. Ainsi, par exemple, les charges de type lamellaires minérales apportent généralement de la douceur et le glissant ; les charges de type sphériques minérales apportent généralement un bon délitage et les charges sphériques organiques ont généralement un rôle structurant et apportent de la douceur.

Parmi les charges de type minérales lamellaires, on peut citer :
- les talcs ou silicates de magnésium hydratés, sous forme de particules de dimensions généralement inférieures à 40 µm ;
- les micas ou aluminosilicates de compositions variées et qui se présentent sous forme d'écailles ayant des dimensions de 2 à 200 µm, de préférence 5-70 µm et une épaisseur de 0,1 à 5 µm, de préférence de 0,2-3 µm, ces micas pouvant être d'origine naturelle (par exemple muscovite, margarite, roscoelite, lipidolite, biotite) ou d'origine synthétique. Ils sont généralement transparents et permettent de conférer à la peau un aspect satiné ;
- les argiles telles que les séricites, qui appartiennent à la même classe chimique et cristalline que la muscovite mais dont les propriétés organoleptiques sont proches du talc ;
- le kaolin ou silicate d'aluminium hydraté, qui se présente sous la forme de particules de formes isotropes ayant des dimensions généralement inférieures à 30µm et qui possèdent de bonnes propriétés d'absorption des corps gras ;
- les nitrures de bore.

Ces charges sont généralement compactables.

Toutefois, parmi ces charges de type lamellaires minérales, certaines sont difficilement compactables. On peut ainsi citer :
- certains talcs, tels que le 'Talc K1' de la société NIPPON ou le 'Talc Extra Steamic OOS' de la société LUZENAC ;
- certaines séricites, telles que la 'Séricite BC282' de la société WHITTAKER ;
- la plupart des micatitanes lorsqu'on les utilise à un fort pourcentage, parmi lesquels on peut citer le mica-nanotitane 'Coverleaf PC 2055M' de la société IKEDA.

Parmi les charges de type lamellaires organiques compactables, on peut citer les poudres de polymères de tétrafluoroéthylène, telles que le 'Fluon' de la société MONTEFLUOS, ou le 'Hostaflonq' de la société HOECHST.

Parmi les charges de type lamellaires organiques difficilement compactables, on peut citer la lauroyl-lysine 'Aminhope LL-11' de la société AJINOMOTO.

Parmi les charges de type sphériques minérales compactables, on peut citer :
- les oxydes de zinc et de titane, généralement utilisés sous la forme de particules ayant des dimensions ne dépassant pas quelques micromètres (ou même inférieures à 1 µm dans le cas de l'oxyde de titane), en particulier des dioxydes de titane sphériques comme le 'SPHERITITAN' de la société IKEDA ; ces oxydes ont un toucher onctueux, un bon pouvoir couvrant et une opacité importante ;
- le carbonate de calcium précipité qui, sous forme de particules de dimensions supérieures à 10 µm, a un toucher onctueux et permet d'obtenir un aspect mat ;
- le carbonate et l'hydrocarbonate de magnésium, qui possèdent, notamment, des propriétés de fixation des parfums ;
- la silice sphérique non poreuse et
- l'hydroxyapatite.

Parmi les charges de type minérales sphériques difficilement compactables, on peut citer :
- les microsphères de silice à porosité ouverte ou, de préférence, les microsphères de silice creuses, telles que les 'SILICA BEADS' de la société MAPRECOS, ces microsphères étant avantageusement imprégnées d'un actif cosmétique, et
- les microcapsules de verre ou de céramique 'MACROLITE' de la société 3M.

Parmi les charges de type organiques sphériques compactables, on peut citer :
- les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium ; ces savons, présents généralement sous la forme de particules ayant des dimensions inférieures à 10 µm, ont un toucher onctueux et facilitent l'adhérence de la poudre à la peau ;
- les poudres de polymères synthétiques non expansés, tels que le polyéthylène, les polyesters (par exemple isophtalate ou téréphtalate de polyéthylène) et les polyamides (par exemple le Nylon), sous la forme de particules ayant des dimensions inférieures à 50 µm, qui possèdent des propriétés absorbantes et permettent de conférer à la peau un aspect velouté ;
- les poudres de polymères synthétiques, réticulés ou non, sphéronisées comme les poudres d'acide polyacrylique ou polyméthacrylique, les poudres de polystyrène réticulé par le divinylbenzène et les poudres de résine de silicone, et
- des poudres de matériaux organiques d'origine naturelle comme l'octenylsuccinate d'amidon vendu sous le nom DRY FLOW PLUS par la société AMYLUM.

Parmi les charges de type organiques sphériques difficilement compactables, on peut citer :
- les microsphères microporeuses de polymères, qui ont une structure analogue à celle d'une éponge ; elles ont, en général, une surface spécifique d'au moins 0,5 m²/g et, en particulier, d'au moins 1 m²/g, ladite surface spécifique n'ayant pas de limite supérieure autre que celle résultant de la possibilité pratique de réaliser des microsphères de porosité très élevée : la surface spécifique peut, par exemple, atteindre 1 000 m²/g ou même davantage. On peut citer les microsphères de polymères acryliques, telles que celles en copolymère d'acrylate réticulé 'Polytrap' de la société DOW CORNING, et celles de polyméthacrylate de méthyle 'MICROPEARL M' ou 'MICROPEARL M 100' de la société SEPPIC ; ces microsphères microporeuses peuvent avantageusement être imprégnées, notamment par des actifs cosmétiques : on peut citer, à cet égard, les microsphères de copolymères de styrènedivinylbenzène vendues sous la dénomination commerciale 'PLASTIC POWDER FPSQ' par la société TOSHIKI, qui sont imprégnées de squalane qui est un actif cosmétique émollient ;
- les microcapsules de polymères ; qui comportent une seule cavité fermée et forment un réservoir, qui peut contenir un liquide, notamment un actif cosmétique ; elles sont préparées par des procédés connus tels que ceux décrits dans le brevet US-A 3 615 972 et EP-A 0 56219. Elles peuvent être réalisées, par exemple, en polymères ou copolymères d'acides, d'amines ou d'esters monomères à insaturation éthylénique, en polymères urée-formaldéhyde, en polymères ou copolymères de chlorure de vinylidène ; à titre d'exemple, on peut citer les microcapsules faites de polymères ou copolymères d'acrylate ou de méthacrylate de méthyie, ou encore de copolymères de chlorure de vinylidène et d'acrylonitrile : parmi ces derniers, on indiquera, notamment, ceux qui contiennent, en poids 20-60% de motifs dérivés de chlorure de vinylidène, 20-60% en poids de motifs dérivés d'acrylonitrile et 0-40% en poids d'autres motifs tels que des motifs dérivés d'un monomère acrylique et/ou styrénique ; on peut également utiliser des polymères ou copolymères acryliques réticulés, par exemple dans le cas de polymères comportant un groupement carboxylique, par des diols servant d'agents réticulants; à titre d'exemple, on peut citer les microcapsules en copolymère de chlorure de vinylidène-acrylonitrile 'EXPANCEL' de la société Casco Nobel, les microcapsules 'Q-MAX' de la société Q-MAX et les microcapsules '3 M' de la société 3M.

Les compositions selon l'invention peuvent contenir en plus une phase grasse. Cette phase grasse peut comprendre des huiles et/ou des cires d'origine animale, végétale. minérale ou synthétique, seules ou en mélanges.

Parmi les huiles utilisables, on peut citer l'huile de vison, l'huile de tortue, l'huile de soja, l'huile de pépins de raisin, l'huile de sésame, l'huile de maïs, l'huile de colza, l'huile de toumesol, l'huile de coton, l'huife d'avocat, l'huile d'olive, l'huile de ricin, l'huile de jojoba, l'huile d'arachide ; les huiles d'hydrocarbures, telles que les huiles de paraffine, le squalane, la vaseline ; les esters gras, tels que le myristate d'isopropyle, le palmitate d'isopropyle, le stéarate de butyle, le laurate d'hexyle, l'isononate d'isononyle, le palmitate de 2-éthyl-hexyle, le laurate de 2-hexyl-décyle, le palmitate de 2-octyl-décyle, le myristate ou le lactate de 2-octy-dodecyle, le succinate de 2-diethyl-hexyle, le malate de diisostéaryle, le triisostéarate de glycérine ou de diglycérine ; les huiles de silicone telles que les polyméthylsiloxanes, les polyméthylphénylsiloxanes, les polysiloxanes modifiés par des acides gras, des alcools gras ou des polyoxyalkylènes, les silicones fluorées, les huiles perfluorées ; les acides gras supérieurs tels que l'acide myristique, l'acide palmitique, l'acide stéarique, l'acide béhénique, l'acide oléique, l'acide linoléique, l'acide linolénique ou l'acide isostéarique ; les alcools gras supérieurs tels que le cétanol, l'alcool stéarylique ou l'alcool oléique.

Parmi les cires utilisables, on peut citer les cires d'abeille, les cires de lanoline et les cires d'insectes de Chine ; les cires de Camauba, de Candelila, d'ouricurry, les cires de fibres de liège, les cires de canne à sucre, les cires du Japon, les cires de jojoba hydrogénées et les huiles hydrogénées telles que l'huile de tournesol hydrogénée, l'huile de ricin hydrogénée, l'huile de coprah hydrogénée et la lanoline hydrogénée ; les paraffines, les cires microcristallines, les cires de Montan et les ozokérites ; les cires de polyéthylène, les cires obtenues par la synthèse de Fisher-Tropsch, les copolymères cireux ainsi que leurs esters, et les cires de silicone telles que les polyalcoxy et polyalkylsiloxanes.

La phase grasse est présente dans des proportions allant, préférentiellement, jusqu'à 20% en poids et plus particulièrement jusqu'à 15% en poids par rapport au poids total de la composition, selon l'application choisie.

La phase grasse peut, en outre, comprendre des additifs tels que des actifs cosmétiques lipophiles et/ou des ingrédients liposolubles généralement utilisés en cosmétique comme les parfums. De préférence, ces additifs peuvent être présents en une quantité allant jusqu'à 20% par rapport au poids total de la phase grasse.

Les compositions selon l'invention peuvent également contenir en plus une ou plusieurs gommes de silicone qui permettent de conférer aux compositions finales des qualités de douceur et de glissant et qui sont généralement difficiles à incorporer de façon homogène à la fois dans les phases anhydres et dans les phases aqueuses.

On utilise de préférence, seule ou en mélange, une gomme de silicone ayant un poids moléculaire inférieur à 1 500 000 telle qu'un polydiméthylsiloxane, un polyphénylsiloxane ou un polyhydroxysiloxane. En particulier, on peut utiliser une gomme de silicone répondant à la formule: 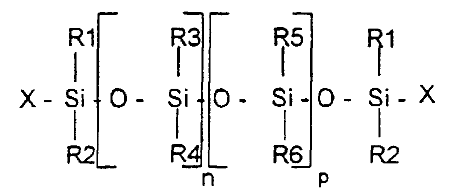 dans laquelle :
R₁, R₂, R₅ et R₆ sont, ensemble ou séparément, un radical alkyle ayant 1 à 6 atomes de carbone,
R₃ et R₄ sont, ensemble ou séparément, un radical alkyle ayant de 1 à 6 atomes de carbone, ou un radical aryle,
X est un radical alkyle ayant de 1 à 6 atomes de carbone, un radical hydroxyle ou un radical vinyle,
n et p étant choisis de manière à conférer à la gomme de silicone une viscosité supérieure à 100 000 mPa.s, de préférence supérieure à 500 000 mPa.s.

De manière générale, n et p peuvent prendre des valeurs de 0 à 5000, de préférence de 0 à 3000.

La gomme de silicone peut être introduite dans la composition telle quelle ou sous forme diluée dans une huile de silicone telle qu'un PDMS (polydiméthylsiloxane).

Comme gomme de silicone utilisable selon l'invention, on peut citer celles pour lesquelles :
. les substituants R₁ à R₆ et X représentent un groupement méthyle, p = 0 et n = 2700, comme celle vendue sous la dénomination SE30 par la société Général Electric,
. les substituants R₁ à R₆ et X représentent un groupement méthyle, p = 0 et n = 2300, comme celle vendue sous la dénomination AK 500000 par la société Waker,
. les substituants R₁ à R₆ représentent un groupement méthyle, le substituant X représente un groupement hydroxyle, p = 0 et n = 2700, en solution à 13 % dans du cyclopentasiloxane, comme celle vendue sous la dénomination Q2-1401 par la société Dow Corning,
. les substituants R₁ à R₆ représentent un groupement méthyle, le substituant X représente un groupement hydroxyle, p = 0 et n = 2700, en solution à 13% dans le diméthicone, comme celle vendue sous la dénomination Q2-1403 par la société Dow Corning,
. les substituants R₁, R₂ R₅, R₆ et X représentent un groupement méthyle, les substituants R₃ et R₄ représentent un groupement aryle tel que le poids moléculaire du composé soit de 600 000, comme celle vendue sous la dénomination 761 par la société Rhône-Poulenc.

Les gommes de silicone sont présentes dans des concentrations allant préférentiellement jusqu'à 40% en poids, selon l'application choisie et plus particulièrement de 5 à 10% en poids par rapport au poids total de la composition.

Les compositions selon l'invention peuvent contenir, outre les charges, des pigments, de préférence en une quantité allant jusqu'à 50% par rapport au poids total de la composition finale. Ces pigments peuvent être choisis parmi les pigments minéraux, les pigments organiques et les pigments nacrés.

Parmi les pigments minéraux, on peut citer, par exemple le dioxyde de titane (rutile ou anatase), éventuellement traité en surface; les oxydes de fer noir, jaune, rouge et brun ; le violet de manganèse; le bleu outremer ; l'oxyde de chrome éventuellement hydraté ; le bleu ferrique.

Parmi les pigments organiques, on peut citer, par exemple, les pigments D & C red, D & C orange, D & C yellow, le noir de carbone, et les laques à base de carmin de cochenille.

Les pigments nacrés peuvent être choisis, notamment, parmi les pigments nacrés blancs tels que le mica recouvert d'oxyde de titane ou l'oxychlorure de bismuth; les pigments nacrés colorés tels que le micatitane avec des oxydes de fer, le micatitane avec du bleu ferrique ou de l'oxyde de chrome, le micatitane avec un pigment organique du type précité, ainsi que les pigments à base d'oxychlorure de bismuth.

Les compositions selon l'invention peuvent également contenir un ou plusieurs tensioactifs non ioniques, anioniques, cationiques ou amphotères, habituellement utilisés en cosmétique. La quantité d'agent tensioactif utilisée est de préférence de 2 à 30% par rapport au poids total de la composition.

Les compositions aqueuses selon l'invention peuvent également contenir en plus des actifs cosmétiques hydrosolubles.

Parmi les actifs cosmétiques, on peut citer les agents antioxydants ou anti-radicaux libres; les agents hydratants ou humectants tels que la glycérine et le collagène ; les agents filtres UV tels que la benzophénone. Ces actifs hydrosolubles peuvent être présents dans la composition finale en une quantité allant jusqu'à 20%, de préférence 5 à 15%. en poids.

La présente invention concerne également un procédé de préparation d'une composition telle que définie précédemment, caractérisé par le fait que celle-ci est obtenue à partir d'un ou plusieurs agent(s) gélifiant(s) hydrosoluble(s) ou hydrophile(s) tels que définis ci-dessus, de la substance cosmétique ou dermatologique et éventuellement des autres ingrédients tels qu'énumérés ci-dessus, en présence d'eau, par mélange, malaxage et extrusion dans un extrudeur bi-vis.

L'extrudeur utilisé pour le procédé de l'invention est choisi parmi les extrudeurs bi-vis tel que celui décrit dans la demande FR 94-00756.

Les matières premières sont introduites, à l'entrée de l'extrudeur bi-vis, dans la zone d'alimentation à température ambiante, de préférence à environ 20°C, puis sont amenées dans la zone de transport à une température, de préférence, à environ 50°C, puis sont malaxées et comprimées dans diverses zones de l'extrudeur maintenues à une température allant, préférentiellement, de 60 à 100°C ; la masse obtenue est transportée vers la sortie de l'extrudeur et extrudée au travers d'une filière.

Pendant la phase de malaxage et de compression, l'agent gélifiant hydrosoluble en contact avec l'eau, forme, après extrusion, un réseau gélifié aqueux constituant la matrice des produits finaux. La masse extrudée sort de la filière sous la forme de boudins de diamètre donné selon la filière utilisée, pouvant être ensuite découpés et mis en forme de bâton, de crayon à mine aqueuse ou de pain solide. D'autres formes peuvent bien entendu être réalisées en choisissant des filières appropriées et des dispositifs de mise en forme des produits finaux adaptés à la forme recherchée.

Les compositions ainsi obtenues ont une matrice constituée d'un réseau gélifié aqueux. Elles peuvent être rendues anhydres par déshydratation du réseau gélifié par un procédé de séchage classique approprié pour donner des produits compacts poudreux tels que des "sticks" poudreux. Les compositions solides anhydres ainsi obtenues peuvent être également réduites en poudre et utilisées de façon classique comme poudre de maquillage sans présentés les inconvénients évoqués précédemment.

Les compositions solides selon l'invention peuvent se présenter sous diverses formes selon l'application choisie. Les formes les plus utilisées sont les bâtons ou "sticks", les crayons ou les pains.

Les compositions selon l'invention, aqueuses ou anhydres, peuvent être des produits pour le maquillage tels que des rouges à lèvres, des fonds de teints, des ombres à paupières sous forme de stick gélifié aqueux ou bien des fards à joues, des fards à paupières ou des anti-cemes sous forme de stick poudreux. Elles peuvent être appliquées directement sur le visage.

Les compositions aqueuses de maquillage de l'invention peuvent être, au moment de l'utilisation, réhydratées partiellement en surface par contact avec de l'eau pour délivrer les substances actives pour le maquillage puis retrouver, après séchage, leur forme initiale sans altération, prêtes pour une autre utilisation dans les mêmes conditions.

Un autre objet de l'invention est donc un procédé de maquillage des lèvres, du visage, du contour des yeux, des joues ou des paupières, caractérisé par le fait que l'on utilise une composition solide aqueuse partiellement réhydratable telle que définie ci-dessus, que l'on mouille celle-ci en surface avec de l'eau et que l'on applique ladite composition partiellement réhydratée sur les lèvres, le visage, le contour des yeux, les joues ou les paupières.

Les compositions aqueuses selon l'invention peuvent être également des produits pour le soin et/ou le conditionnement et/ou l'hygiène de la peau, des muqueuses, du cuir chevelu ou des cheveux. Elles sont généralement sous forme de stick, de crayon ou de pain. Elles peuvent en particulier être appliquées sur les matières kératiniques, au moment de l'emploi, par simple hydratation partielle en surface en contact avec de l'eau pour délivrer les substances actives et retrouver, après séchage, leur forme initiale sans altération, prêtes pour une autre utilisation dans les mêmes conditions.

Parmi les produits de soin, de conditionnement ou d'hygiène envisageables, on peut mentionner, par exemple, en capillaire : des gels solides de coiffage en forme de stick ; en soin de la peau : des démaquillants, des hydratants, des amincissants en forme de stick ou de pain, des produits pour le soin des lèvres en forme de stick ou de crayon ; en hygiène : des shampooings, des produits pour le rasage, le bain ou la douche, des déodorants, en forme de stick ou de pain.

Un autre objet de l'invention consiste en un procédé de traitement cosmétique pour le soin et/ou conditionnement et/ou l'hygiène de la peau, des cheveux, du cuir chevelu ou des muqueuses, caractérisé par le fait que l'on utilise une composition solide aqueuse telle que définie ci-dessus, que l'on mouille celle-ci en surface avec de l'eau et que l'on applique ladite composition partiellement réhydratée sur la peau, les cheveux, le cuir chevelu ou les muqueuses.

Un autre objet de l'invention porte sur sur l'utilisation d'une composition solide anhydre que définie ci-dessus, comme poudre de maquillage.

Bien entendu, l'homme de l'art veillera à choisir ce ou ces éventuels composés complémentaires et/ou leurs quantités de manière telle que les propriétés avantageuses attachés intrinsèquement à la composition conforme à l'invention ne soient pas, ou substantiellement pas, altérées par la ou les adjonctions envisagées.

L' exemple qui suit sert à illustrer l'invention.

### EXEMPLE

### Bâtons de rouge à lèvres à réhydrater partiellement

Le produit final a pour formulation suivante :
- Gomme de caroube (gélifiant hydrosoluble) 30,0% en poids
- Pigments hydrosolubles 10,0% en poids
- Particules expansées de copolymère 1,0% en poids
   de chlorure de vinylidène/acrylonitrile/
   méthacrylate de méthyle vendu sous le nom
   EXPANCEL 550DE par la Société CASCO NOBEL
- Silice vendue sous le nom SB700 10,0% en poids
   par la Société MAPRECOS
- Conservateur 0,5% en poids
- Eau qsp 100,0% en poids

### MODE OPERATOIRE :

Les "sticks" sont obtenus par extrusion dans un extrudeur bi-vis. Les matières premières sont introduites à l'entrée de l'extrudeur à une température de 30°C. Elles sont ensuite amenées dans la zone de transport à une température de 50°C, puis sont malaxées et comprimées dans diverses zones de l'extrudeur maintenues à 100°C. La masse ainsi malaxée et comprimée, est transportée vers la sortie de l'extrudeur et extrudée au travers d'une filière de 1 cm de diamètre. La vitesse de rotation des vis est de 500 tours/minute. Les boudins obtenus à la sortie de la filière sont réduits sous forme de bâtonnets de 3 cm de longueur au moyen d'un dispositif de découpe en sortie de l'extrudeur.

## Revendications

1. Composition à usage cosmétique ou dermatologique, **caractérisée par le fait qu'**elle est constituée par un gel rigide présentant une résistance à la compression supérieure ou égale à 50 grammes, à température ambiante, ledit gel contenant au moins 20% en poids d'au moins un agent gélifiant hydrosoluble ou hydrophile par rapport au poids total de la composition et au moins une substance cosmétique ou dermatologique, ledit gel étant susceptible d'être obtenu à partir dudit agent gélifiant et de ladite substance cosmétique ou dermatologique en présence d'eau, par mélange, malaxage, compression et extrusion dans un extrudeur bi-vis.

2. Composition selon la revendication 1, **caractérisée par le fait que** le gel rigide est susceptible d'être obtenu par un procédé dans lequel ledit agent gélifiant, ladite substance cosmétique ou dermatologique et l'eau sont introduits, à l'entrée de l'extrudeur, à température ambiante puis sont amenés dans la zone de transport à température d'environ 50°C, puis sont malaxées et comprimées dans diverses zones de l'extrudeur maintenues à une température allant de 60 à 100°C; la masse obtenue est transportée vers la sortie de l'extrudeur et extrudée au travers d'une filière.

3. Composition selon l'une quelconque des revendications précédentes, **caractérisée par le fait que** le gel rigide est susceptible d'être rendu anhydre par déshydratation du réseau gélifié aqueux par un procédé de séchage classique.

4. Composition selon l'une quelconque des revendications précédentes, **caractérisée par le fait que** l'agent gélifiant hydrosoluble ou hydrophile est choisi dans le groupe formé par
- les extraits d'algue ;
- les extraits de graines ;
- les exsudats de plantes ;
- les exsudats de microorganismes ;
- les extraits de fruits ;
- les agents gélifiants d'origine animale ;
- les polymères synthétiques gélifiants hydrosolubles ;
- les dérivés du silicium.

5. Composition selon l'une des revendications précédentes, **caractérisée par le fait que** l'agent hydrosoluble gélifiant est choisi dans le groupe formé par l'agar-agar, les carraghénanes, les alginates, la gomme de caroube, la gomme de guar, la gomme arabique, la gomme karaya, la gomme adragante, la gomme de gatty, la gomme de xanthane, la cellulose ou ses dérivés, les celluloses modifiées, les pectines ; la gélatine, les caséïnates, les acides polyacryliques réticulés, les hectorites synthétiques, les silicates d'aluminium et de magnésium.

6. Composition selon l'une quelconque des revendications précédentes, **caractérisée par le fait que** l'agent gélifiant hydrosoluble ou hydrophile est présent dans des concentrations allant de 20 à 80% en poids par rapport au poids total de la composition.

7. Composition selon l'une quelconque des revendications précédentes, contenant au moins une charge minérale et/ou une charge organique.

8. Composition selon la revendication 7, **caractérisée par le fait qu'**elle contient jusqu'à 80% en poids de charge par rapport au poids total de la composition.

9. Composition selon l'une quelconque des revendications 7 à 8, **caractérisée par le fait que** :
- lorsque la ou les charges ont une densité inférieure à 0,1 g.cm⁻³, elles sont présentes jusqu'à 40% en poids par rapport au poids total de la composition et
- lorsque la ou les charges ont une densité supérieure à 0,5 g.cm³, elles sont présentes à raison de 2 à 80% en poids par rapport au poids total de la composition.

10. Composition selon l'une quelconque des revendications 7 à 9, **caractérisée par le fait que** la ou les charges sont choisies parmi les charges minérales ou organiques, de structure lamellaire ou sphérique, compactables ou difficilement compactables et leurs mélanges.

11. Composition selon la revendication 10, dans laquelle les charges de type minérales lamellaires sont choisies parmi les talcs ou silicates de magnésium hydratés, les micas ou aluminosilicates, les argiles telles que les séricites, le kaolin ou silicate d'aluminium hydraté, les nitrures de bore, les micatitanes.

12. Composition selon la revendication 10, dans laquelle les charges de type sphériques minérales sont choisies parmi les oxydes de zinc et de titane, le carbonate de calcium précipité, le carbonate et l'hydro-carbonate de magnésium, la silice sphérique non poreuse, l'hydroxyapatite, les microsphères de silice à porosité ouverte ou creuses, éventuellement imprégnées d'un actif cosmétique et les microcapsules de verre ou de céramique.

13. Composition selon la revendication 10, dans laquelle les charges de type organiques sphériques sont choisies parmi les savons métalliques dérivés d'acides organiques carboxyliques ayant de 8 à 22 atomes de carbone, tels que le stéarate de zinc, de magnésium ou de lithium, le laurate de zinc, le myristate de magnésium ; les poudres de polymères synthétiques non expansés; les poudres de polymères synthétiques réticulés ou non, sphéronisées; les poudres de matériaux organiques d'origine naturelle, les microsphères microporeuses de polymères, éventuellement imprégnées par des actifs cosmétiques ; les microcapsules de polymères éventuellement réticulés.

14. Composition selon l'une quelconque des revendications précédentes, **caractérisée par le fait qu'**elle contient en plus une phase grasse.

15. Composition selon la revendication 14, comprenant jusqu'à 20% en poids de phase grasse et de préférence jusqu'à 15% en poids par rapport au poids total de la composition.

16. Composition selon l'une des revendications 14 à 15, dans laquelle la phase grasse comprend des huiles et/ou des cires d'origine animale, végétale, minérale ou synthétique, seules ou en mélanges.

17. Composition selon l'une quelconque des revendications précédentes, **caractérisée par** le fait quel contient des adjuvants choisis parmi les pigments, les tensio-actifs, les actifs liposolubles, les additifs liposolubles habituellement utilisés en cosmétique, les antioxydants, les anti-radicaux libres, les hydratants, les humectants, les filtres solaires.

18. Composition selon l'une quelconque des revendications précédentes, comprenant au moins une gomme de silicone.

19. Composition selon la revendication 18, dans laquelle la gomme de silicone est présente en une quantité allant jusqu'à 40%, de préférence de 5 à 10% par rapport au poids de la composition finale.

20. Composition selon l'une des revendications 18 à 19, dans laquelle la gomme de silicone est choisie parmi les gommes de silicone ayant un poids moléculaire inférieur à 1 500 000 telles qu'un polydiméthylsiloxane, un polyphénylsiloxane ou un polyhydroxysiloxane, et/ou parmi les gommes répondant à la formule : 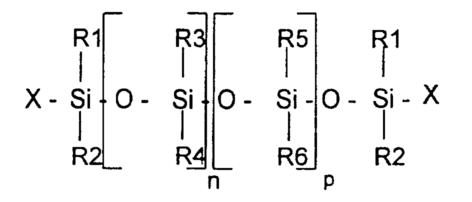 dans laquelle :
- R₁, R₂, R₅ et R₆ sont, ensemble ou séparément, un radical alkyle ayant 1 à 6 atomes de carbone,
- R₃ et R₄ sont, ensemble ou séparément, un radical alkyle ayant de 1 à 6 atomes de carbone, ou un radical aryle,
- X est un radical alkyle ayant de 1 à 6 atomes de carbone, un radical hydroxyle ou un radical vinyle,
- n et p étant choisis de manière à conférer à la gomme de silicone une viscosité supérieure à 100 000 mPa.s, de préférence supérieure à 500 000 mPa.s.

21. Composition selon l'une quelconque des revendications précédentes, **caractérisée par le fait qu'**elle se présente sous forme de bâton ou "stick", de crayon ou de pain.

22. Composition selon l'une quelconque des revendications 1-2 et 4-21, **caractérisée par le fait qu'**elle est un produit pour le soin et/ou le conditionnement et/ou l'hygjène de la peau, des muqueuses, du cuir chevelu bu des cheveux.

23. Composition selon l'une quelconque des revendications 1-21, **caractérisée par le fait qu'**elle est un produit pour le maquillage.

24. Poudre de maquillage, **caractérisée par le fait qu'**elle est constituée d'une composition anhydre avec une matrice constituée d'un réseau gélifié déshydraté telle que définie selon l'une quelconque des revendications 3 à 21 ayant été réduite en poudre.

25. Utilisation comme produit cosmétique d'un gel rigide présentant une résistance à la compression supérieure ou égale à 50 grammes, à température ambiante, et contenant au moins 20% en poids d'au moins un agent gélifiant hydrosoluble ou hydrophile, ledit gel étant susceptible d'être obtenu à partir dudit agent gélifiant en présence d'eau, par mélange, malaxage, compression et extrusion dans un extrudeur bi-vis.

26. Utilisation selon la revendication 25, **caractérisée par le fait que** le gel rigide est susceptible d'être obtenu par un procédé dans lequel l'agent gélifiant et l'eau sont introduits, à l'entrée de l'extrudeur, à température ambiante puis sont amenées dans la zone de transport à température d'environ 50°C, puis sont malaxées et comprimées dans diverses zones de l'extrudeur maintenues à une température allant de 60 à 100°C; la masse obtenue est transportée vers la sortie de l'extrudeur et extrudée au travers d'une filière.

27. Utilisation selon l'une quelconque des revendications 25 à 26, **caractérisée par le fait que** le gel rigide est susceptible d'être rendu anhydre par déshydratation du réseau gélifié aqueux par un procédé de séchage classique.

28. Utilisation selon l'une quelconque des revendications 25 à 27, **caractérisée par le fait que** l'agent gélifiant hydrosoluble ou hydrophile est choisi dans le groupe formé par :
- les extraits d'algue ;
- les extraits de graines ;
- les exsudats de plantes ;
- les exsudats de microorganismes ;
- les extraits de fruits ;
- les agents gélifiants d'origine animale ;
- les polymères synthétiques gélifiants hydrosolubles ;
- les dérivés du silicium.

29. Utilisation selon la revendication 28, **caractérisée par le fait que** l'agent hydrosoluble gélifiant est choisi dans le groupe formé par l'agar-agar, les carraghénanes, les alginates, la gomme de caroube, la gomme de guar, la gomme arabique, la gomme karaya, la gomme adragante, la gomme de gatty, la gomme de xanthane, la cellulose ou ses dérivés, les celluloses modifiées, les pectines ; la gélatine, les caséïnates, les acides polyacryliques réticulés, les hectorites synthétiques, les silicates d'aluminium et de magnésium.

30. Utilisation selon l'une quelconque des revendications 25 à 29, **caractérisée par le fait que** l'agent gélifiant hydrosoluble ou hydrophile est présent dans des concentrations allant de 20 à 80% en poids par rapport au poids total de la composition.

31. Procédé de préparation de la composition sous forme de gel rigide telle que définie dans l'une quelconque des revendications 1 à 24, **caractérisé par le fait qu'**elle est obtenue à partir d'au moins un agent gélifiant hydrosoluble ou hydrophile, de la substance dermatologique ou cosmétique et éventuellement des autres constituants tels que définis dans les revendications précédentes, en présence d'eau par mélange, malaxage, compression et extrusion dans un extrudeur bi-vis puis on effectue éventuellement une déshydratation du gel rigide par un procédé classique de séchage.

32. Procédé selon la revendication 31, **caractérisé par le fait que** les matières premières sont introduites, à l'entrée de l'extrudeur, à température ambiante puis sont amenées dans la zone de transport à température d'environ 50°C, puis sont malaxées et comprimées dans diverses zones de l'extrudeur maintenues à une température allant de 60 à 100°C; la masse obtenue est transportée vers la sortie de l'extrudeur et extrudée au travers d'une filière.

33. Procédé de traitement cosmétique pour le soin et/ou conditionnement et/ou l'hygiène de la peau, des cheveux, du cuir chevelu ou des muqueuses, **caractérisé par le fait que** l'on utilise une composition solide aqueuse selon l'une quelconque des revendications 1-2 et 4-21, que l'on mouille celle-ci en surface avec de l'eau et que l'on applique ladite composition partiellement réhydratée sur la peau, les cheveux, le cuir chevelu ou les muqueuses.

34. Procédé de maquillage des lèvres, du visage ou des paupières, **caractérisé par le fait que** l'on utilise une composition solide aqueuse selon l'une quelconque des revendications 1-2 et 4-21, que l'on mouille celle-ci en surface avec de l'eau et que l'on applique ladite composition partiellement réhydratée sur les lèvres, le visage ou les paupières.

## Patentansprüche

1. Zusammensetzung zur kosmetischen oder dermatologischen Verwendung, **dadurch gekennzeichnet, daß** sie aus einem festen Gel besteht, das bei Raumtemperatur eine Druckfestigkeit von mindestens 50 g aufweist, wobei das Gel mindestens 20 Gew.-% mindestens eines wasserlöslichen oder hydrophilen Gelbildners, bezogen auf das Gesamtgewicht der Zusammensetzung, und mindestens eine kosmetische oder dermatologische Substanz enthält, wobei das Gel ausgehend von dem Gelbildner und der kosmetischen oder dermatologischen Substanz in Gegenwart von Wasser durch Mischen, Kneten, Verdichten und Extrudieren in einem Zweischneckenextruder hergestellt werden kann.

2. Zusammensetzung nach Anspruch 1, **dadurch gekennzeichnet, daß** das feste Gel nach einem Verfahren hergestellt werden kann, nach dem der Gelbildner, die kosmetische oder dermatologische Substanz und Wasser bei Raumtemperatur am Einlaß des Extruders zugegeben werden, anschließend in der Förderzone auf eine Temperatur von etwa 50 °C erwärmt werden und dann in den unterschiedlichen Zonen des Extruders, die bei einer Temperatur von 60 bis 100 °C gehalten werden, geknetet und verdichtet werden, und die erhaltene Masse zum Auslaß des Extruders gefördert und durch eine Düse extrudiert wird.

3. Zusammensetzung nach einem der vorhergehenden Ansprüche, **dadurch gekennzeichnet, daß** das feste Gel nach einem herkömmlichen Trocknungsverfahren durch Dehydratisierung des wäßrigen gelierten Netzwerkes wasserfrei gemacht werden kann.

4. Zusammensetzung nach einem der vorhergehenden Ansprüche, **dadurch gekennzeichnet, daß** der wasserlösliche oder hydrophile Gelbildner ausgewählt ist unter:
- Algenextrakten;
- Samenextrakten;
- Pflanzenexsudaten;
- Exsudaten aus Mikroorganismen;
- Fruchtextrakten;
- Gelbildnern tierischer Herkunft;
- wasserlöslichen synthetischen gelierenden Polymeren; und
- Siliciumderivaten.

5. Zusammensetzung nach einem der vorhergehenden Ansprüche, **dadurch gekennzeichnet, daß** der wasserlösliche Gelbildner unter Agar-Agar, Carrageenanen, Alginaten, Johannisbrot-Kemmehl, Guar-Gummi, Gummi arabicum, Karaya-Gummi, Tragant, Ghatti Gummi, Xanthangummi, Cellulose und ihren Derivaten, modifizierten Cellulosen, Pektinen, Gelatine, Caseinaten, vemetzten Polyacrylsäuren, synthetischen Hectoriten und Aluminiummagnesiumsilicaten ausgewählt ist.

6. Zusammensetzung nach einem der vorhergehenden Ansprüche, **dadurch gekennzeichnet, daß** der wasserlösliche oder hydrophile Gelbildner in Konzentrationen von 20 bis 80 Gew.-%, bezogen auf das Gesamtgewicht der Zusammensetzung, vorliegt.

7. Zusammensetzung nach einem der vorhergehenden Ansprüche, die mindestens einen anorganischen und/oder organischen Füllstoff enthält.

8. Zusammensetzung nach Anspruch 7, **dadurch gekennzeichnet, daß** sie bis zu 80 Gew.-% Füllstoff, bezogen auf das Gesamtgewicht der Zusammensetzung, enthält.

9. Zusammensetzung nach einem der Ansprüche 7 oder 8, **dadurch gekennzeichnet, daß**:
- der oder die Füllstoffe in einem Anteil von bis zu 40 Gew.-%, bezogen auf das Gesamtgewicht der Zusammensetzung, enthalten sind, wenn sie eine Dichte unter 0,1 g·cm⁻³ aufweisen, und
- der oder die Füllstoffe in einem Anteil von 2 bis 80 Gew.-%, bezogen auf das Gesamtgewicht der Zusammensetzung, enthalten sind, wenn sie eine Dichte über 0,5 g·cm⁻³ aufweisen.

10. Zusammensetzung nach einem der Ansprüche 7 bis 9, **dadurch gekennzeichnet, daß** der oder die Füllstoffe unter den anorganischen oder organischen Füllstoffen lamellarer oder kugelförmiger Struktur, die verdichtet werden können oder nur schwer zu verdichten sind, und deren Gemischen ausgewählt sind.

11. Zusammensetzung nach Anspruch 10, wobei die Füllstoffe vom Typ der lamellaren anorganischen Füllstoffe unter Talken oder hydratisierten Magnesiumsilicaten, Glimmern oder Aluminosilicaten, Tonen, wie Sericiten, Kaolin oder hydratisiertem Aluminiumsilicat, Bornitriden und Titanglimmern ausgewählt sind.

12. Zusammensetzung nach Anspruch 10, wobei die Füllstoffe vom Typ der anorganischen kugelförmigen Füllstoffe unter Zinkoxid und Titanoxid, gefälltem Calciumcarbonat, Magnesiumcarbonat, Magnesiumhydrogencarbonat, nicht porösem kugelförmigen Siliciumdioxid, Hydroxyapatit, offenporigen oder hohlen Mikrokugeln aus Siliciumdioxid, die gegebenenfalls mit einem kosmetischen Wirkstoff imprägniert sind, und Mikrokapseln aus Glas oder Keramik ausgewählt sind.

13. Zusammensetzung nach Anspruch 10, wobei die Füllstoffe vom Typ der organischen kugelförmigen Füllstoffe unter den Metallseifen, die von organischen Carbonsäuren mit 8 bis 22 Kohlenstoffatomen abgeleitet sind, beispielsweise Zinkstearat, Magnesium stearat, Lithiumstearat, Zinklaurat und Magnesiummyristat; Pulvern von nicht expandierten synthetischen Polymeren; Pulvern von vernetzten oder nicht vernetzten synthetischen Polymeren, die zu Kügelchen geformt wurden; Pulvern organischer Materialien natürlicher Herkunft, mikroporösen Mikrokugeln von Polymeren, die gegebenenfalls mit kosmetischen Wirkstoffen imprägniert sind; und Mikrokapseln von gegebenenfalls vernetzten Polymeren ausgewählt sind.

14. Zusammensetzung nach einem der vorhergehenden Ansprüche, **dadurch gekennzeichnet, daß** sie ferner eine Fettphase enthält.

15. Zusammensetzung nach Anspruch 14, die bis zu 20 Gew,-% Fettphase und vorzugsweise bis zu 15 Gew.-%, bezogen auf das Gesamtgewicht der Zusammensetzung, enthält.

16. Zusammensetzung nach einem der Ansprüche 14 bis 15, wobei die Fettphase Öle und/oder Wachse tierischer, pflanzlicher, mineralischer oder synthetischer Herkunft einzeln oder im Gemisch enthält.

17. Zusammensetzung nach einem der vorhergehenden Ansprüche, **dadurch gekennzeichnet, daß** sie Zusatzstoffe enthält, die unter den Pigmenten, grenzflächenaktiven Stoffen, fettlöslichen Wirkstoffen, fettlöslichen Zusatzstoffen, die üblicherweise in der Kosmetik verwendet werden, Antioxidantien, Mitteln gegen freie Radikale, Hydratisierungsmittel, Feuchthaltemitteln und Sonnenschutzfiltern ausgewählt sind.

18. Zusammensetzung nach einem der vorhergehenden Ansprüche, die mindestens einen Silicongummi enthalten.

19. Zusammensetzung nach Anspruch 18, wobei der Silicongummi in einer Menge von bis zu 40 % und vorzugsweise 5 bis 10 %, bezogen auf das Gewicht der fertigen Zusammensetzung, vorliegt.

20. Zusammensetzung nach einem der Ansprüche 18 bis 19, wobei der Silicongummi unter den Silicongummis mit einem Molekulargewicht unter 1 500 000, beispielsweise einem Polydimethylsiloxan, einem Polyphenylsiloxan oder einem Polyhydroxysiloxan, und/oder unter den Gummis der folgenden Fonnel ausgewählt ist: 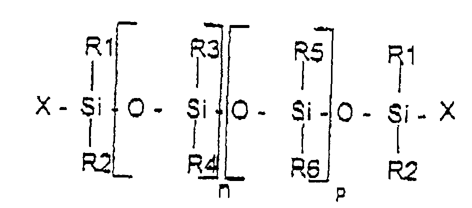 worin bedeuten:
- die Gruppen R₁, R₂, R₅ und R₆ gemeinsam oder unabhängig voneinander eine Alkylgruppe mit 1 bis 6 Kohlenstoffatomen,
- die Gruppen R₃ und R₄ gemeinsam oder unabhängig voneinander eine Alkylgruppe mit 1 bis 6 Kohlenstoffatomen oder eine Arylgruppe,
- X eine Alkylgruppe mit 1 bis 6 Kohlenstoffatomen, eine Hydroxygruppe oder eine Vinylgruppe,
- wobei n und p so ausgewählt sind, daß der Silicongummi eine Viskosität über 100 000 mPa · s und vorzugsweise über 500 000 mPa · s aufweist.

21. Zusammensetzung nach einem der vorhergehenden Ansprüche, **dadurch gekennzeichnet, daß** sie als Stift oder "Stick", Minenstift oder Riegel vorliegt.

22. Zusammensetzung nach einem der Ansprüche 1 bis 2 und 4 bis 21, **dadurch gekennzeichnet, daß** sie eine Produkt zur Pflege und/oder zum Konditionieren und/oder zur Hygiene der Haut, der Schleimhäute, der Kopfhaut oder der Haare ist.

23. Zusammensetzung nach einem der Ansprüche 1 bis 21, **dadurch gekennzeichnet, daß** es sich um ein Schminkprodukt handelt.

24. Pulver zum Schminken, **dadurch gekennzeichnet, daß** es aus einer wasserfreien Zusammensetzung nach einem der Ansprüche 3 bis 21 besteht, die eine Matrix aufweist, welche aus einem dehydratisierten gelierten Netzwerk gebildet wird, und die zu einem Pulver zerkleinert wurde.

25. Verwendung eines festen Gels als kosmetisches Produkt, welches bei Raumtemperatur eine Druckfestigkeit von mindestens 50 g aufweist und mindestens 20 Gew.-% mindestens eines wasserlöslichen oder hydrophilen Gelbildners enthält, wobei das Gel ausgehend von dem Gelbildner in Gegenwart von Wasser durch Mischen, Kneten, Verdichten und Extrudieren in einem Zweischneckenextruder hergestellt werden kann.

26. Verwendung nach Anspruch 25, **dadurch gekennzeichnet, daß** das feste Gel nach einem Verfahren hergestellt werden kann, wonach der Gelbildner und Wasser bei Raumtemperatur am Einlaß des Extruders zugegeben werden, anschließend in der Förderzone auf eine Temperatur von etwa 50 °C erwärmt werden und dann in den unterschiedlichen Zonen des Extruders, die bei einer Temperatur von 60 bis 100 °C gehalten werden, geknetet und verdichtet werden, und die erhaltene Masse zum Auslaß des Extruders gefördert und durch eine Düse extrudiert wird.

27. Verwendung nach einem der Ansprüche 25 bis 26, **dadurch gekennzeichnet, daß** das feste Gel nach einem herkömmlichen Trocknungsverfahren durch Dehydratisierung des wäßrigen gelierten Netzwerkes wasserfrei gemacht werden kann.

28. Verwendung nach einem der Ansprüche 25 bis 27, **dadurch gekennzeichnet, daß** der wasserlösliche oder hydrophile Gelbildner ausgewählt ist unter:
- Algenextrakten;
- Samenextrakten;
- Pflanzenexsudaten;
- Exsudaten aus Mikroorganismen;
- Fruchtextrakten;
- Gelbildnern tierischer Herkunft;
- wasserlöslichen synthetischen gelierenden Polymeren; und
- Siliciumderivaten.

29. Verwendung nach Anspruch 28, **dadurch gekennzeichnet, daß** der wasserlösliche Gelbildner unter Agar-Agar, Carrageenanen, Alginaten, Johannisbrot-Kernmehl, Guar-Gummi, Gummi arabicum, Karaya-Gummi, Tragant, Ghatti Gummi, Xanthangummi, Cellulose und ihren Derivaten, modifizierten Cellulosen, Pektinen, Gelatine, Caseinaten, vemetzten Polyacrylsäuren, synthetischen Hectoriten und Aluminiummagnesiumsilicaten ausgewählt ist.

30. Verwendung nach einem der Ansprüche 25 bis 29, **dadurch gekennzeichnet, daß** der wasserlösliche oder hydrophile Gelbildner in Konzentrationen von 20 bis 80 Gew.-%, bezogen auf das Gesamtgewicht der Zusammensetzung, vorliegt.

31. Verfahren zur Herstellung einer Zusammensetzung in Form eines festen Gels nach einem der Ansprüche 1 bis 24, **dadurch gekennzeichnet, daß** sie ausgehend von mindestens einem wasserlöslichen oder hydrophilen Gelbildner, einer dermatologischen oder kosmetischen Substanz und gegebenenfalls weiteren Bestandteilen, beispielsweise in den vorhergehenden Ansprüche definierten Bestandteilen, in Gegenwart von Wasser durch Mischen, Kneten, Verdichten und Extrudieren in einem Zweischneckenextruder hergestellt werden kann, wobei das feste Gel anschließend gegebenenfalls nach einem herkömmlichen Trocknungsverfahren dehydratisiert wird.

32. Verfahren nach Anspruch 31, **dadurch gekennzeichnet, daß** die Ausgangssubstanzen am Einlaß des Extruders bei Umgebungstemperatur zugegeben werden, anschließend in der Förderzone auf eine Temperatur von etwa 50 °C erwärmt werden und dann in den unterschiedlichen Bereichen des Extruders, die bei einer Temperatur von 60 bis 100 °C gehalten werden, geknetet und komprimiert werden und die erhaltene Masse zum Auslaß des Extruders gefördert und durch eine Düse extrudiert wird.

33. Verfahren zur kosmetischen Behandlung zur Pflege und/oder zum Konditionieren und/oder zur Hygiene der Haut, der Haare, der Kopfhaut oder der Schleimhäute, **dadurch gekennzeichnet, daß** eine wäßrige feste Zusammensetzung nach einem der Ansprüche 1 bis 2 und 4 bis 21 verwendet wird, diese an der Oberfläche mit Wasser befellchtet wird und die zum Teil rehydratisierte Zusammensetzung auf die Haut, die Haare, die Kopfhaut oder die Schleimhäute aufgetragen wird.

34. Verfahren zum Schminken der Lippen, des Gesichts oder der Lider, **dadurch gekennzeichnet, daß** eine wäßrige feste Zusammensetzung nach einem der Ansprüche 1 bis 2 und 4 bis 21 verwendet wird, diese an der Oberfläche mit Wasser befeuchtet wird und die zum Teil rehydratisierte Zusammensetzung auf die Lippen, das Gesicht oder die Lider aufgetragen wird.

## Claims

1. Composition for cosmetic or dermatological use, **characterized in that** it consists of a rigid gel having a compression strength of greater than or equal to 50 grams, at room temperature, the said gel containing at least 20% by weight of at least one water-soluble or hydrophilic gelling agent relative to the total weight of the composition and at least one cosmetic or dermatological substance, it being posible for the said gel to be obtained from the said gelling agent and from the said cosmetic or dermatological substance in the presence of water, by mixing, blending, compression and extrusion in a twin-screw extruder.

2. Composition according to Claim 1, **characterized in that** the rigid gel may be obtained by a process in which the said gelling agent, the said cosmetic or dermatological substance and the water are introduced, at the extruder inlet, at room temperature, and are then brought into the transportation zone at a temperature of about 50°C, after which they are blended and compressed in various zones of the extruder maintained at a temperature ranging from 60 to 100°C; the mass obtained is transported to the extruder outlet and extruded through a die.

3. Composition according to any one of the preceding claims, **characterized in that** the rigid gel may be made anhydrous by dehydration of the aqueous gelied network by a standard drying process.

4. Composition according to any one of the prececeding claims, **characterized in that** the water-soluble or hydrophilic gelling agent is chosen from the group formed by:
- alga extracts;
- seed extracts;
- plant exudates;
- microorganism exudates;
- fruit extracts;
- gelling agents of animal origin;
- water-soluble gelling synthetic polymers;
- silicon derivatives.

5. Composition according to one of the preceding claims, **characterized in that**. the water-soluble gelling agent is chosen from the group formed by agar-agar, carageenans, alginates, carob gum, guar gum, gum arabic, karaya gum, gum tragacanth, ghatti gum, xanthan gum, cellulose or derivatives thereof, modified celluloses, pectins; gelatin, caseinates, crosslinked polyacrylic acids, synthetic hectorites, and aluminium and magnesium silicates.

6. Composition according to any one of the preceding claims, **characterized in that** the water-soluble or hydrophilic gelling agent is present in concentrations ranging from 20 to 80% by weight relative to the total weight of the composition.

7. Composition according to any one of the preceding claims, containing at least one inorganic filler and/or one organic filler.

8. Composition according to Claim 7, **characterized in that** it contains up to 80% by weight of filler relative to the total weight of the composition.

9. Composition according to either of Claims 7 and 8, **characterized in that**:
- when the filler or fillers have a density of less than 0.1 g.cm⁻³, they are present at up to 40% by weight relative to the total weight of the composition, and
- when the filler or fillers have a density of greater than 0.5 g.cm⁻³, they are present in a proportion of from 2 to 80% by weight relative to the total weight of the composition.

10. Composition according to any one of Claims 7 to 9, **characterized in that** the filler or fillers are chosen from inorganic or organic fillers of lamellar or spherical structure, which are compactable or not readily compacted, and mixtures thereof.

11. Composition according to Claim 10, in which the fillers of inorganic lamellar type are chosen from talcs or hydrated magnesium silicates, micas or aluminosilicates, clays such as sericites, kaolin or hydrated aluminium silicate, boron nitrides and titanium micas.

12. Composition according to Claim 10, in which the fillers of inorganic spherical type are chosen from oxides of zinc and of titanium, precipitated calcium carbonate, magnesium carbonate and hydrocarbonate, nonporous spherical silica, hydroxyapatite, silica microspheres with open or hollow porosity, which may be impregnated with a cosmetic active agent, and glass or ceramic microcapsules.

13. Composition according to Claim 10, in which the fillers of organic spherical type are chosen from metal soaps derived from organic carboxylic acids having from 8 to 22 carbon atoms, such as zinc, magnesium or lithium stearate, zinc laurate and magnesium myristate; non-expanded synthetic polymer powders; spheronized, crosslinked or non-crosslinked synthetic polymer powders; powders of organic materials of natural origin, microporous polymer microspheres which may be impregnated with cosmetic active agents; polymer microcapsules which are optionally crosslinked.

14. Composition according to any one of the preceding claims, **characterized in that** it also contains a fatty phase.

15. Composition according to Claim 14, comprising up to 20% by weight of fatty phase and preferably up to 15% by weight relative to the total weight of the composition.

16. Composition according to either of Claims 14 and 15, in which the fatty phase comprises oils and/or waxes of animal, plant, inorganic or synthetic origin, alone or as mixtures.

17. Composition according to any one of the preceding claims, **characterized in that** it contains adjuvants chosen from pigments, surfactants, liposoluble active agents, liposoluble additives usually used in cosmetics, antioxidants, anti-free-radical agents, moisturizers, wetting agents and sunscreens.

18. Composition according to any one of the preceding claims, comprising at least one silicone gum.

19. Composition according to Claim 18, in which the silicone gum is present in an amount ranging up to 40%, preferably from 5 to 10%, relative to the weight of the final composition.

20. Composition according to either of Claims 18 and 19, in which the silicone gum is chosen from silicone gums having a molecular weight of less than 1,500,000, such as a polydimethylsiloxane, a polyphenylsiloxane or a polyhydroxysiloxane, and/or from gums corresponding to the formula: 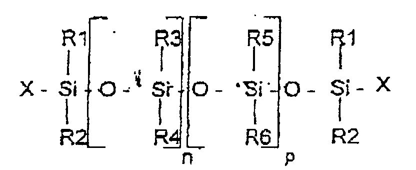 in which:
- R₁, R₂, R₅ and R₆ are, together or separately, an alkyl radical having 1 to 6 carbon atoms,
- R₃ and R₄ are, together or separately, an alkyl radical having from 1 to 6 carbon atoms, or an aryl radical,
- X is an alkyl radical having from 1 to 6 carbon atoms, a hydroxyl radical or a vinyl radical,
- n and p being chosen so as to impart a viscosity of greater than 100,000 mPa.s, preferably greater than 500,000 mPa.s, to the silicone gum.

21. Composition according to any one of the preceding claims, **characterized in that** it is in the form of a stick, a pencil or a cake.

22. Composition according to any one of Claims 1-2 and 4-21, **characterized in that** it is a care and/or conditioning and/or hygiene product for the skin, the mucous membranes, the scalp or the hair.

23. Composition according to any one of Claims 1-21, **characterized in that** it is a make-up product.

24. Make-up powder, **characterized in that** it consists of an anhydrous composition with a matrix consisting of a dehydrated gelled network, as defined according to any one of Claims 3 to 21, this composition having been reduced to a powder.

25. Use, as cosmetic product, of a rigid gel having a compression strength of greater than or equal to 50 grams, at room temperature, and containing at least 20% by weight of at least one water-soluble or hydrophilic gelling agent, it being possible for the said gel to be obtained from the said gelling agent in the presence of water, by mixing, blending, compression and extrusion in a twin-screw extruder.

26. Use according to Claim 25, **characterized in that** the rigid gel may be obtained by a process in which the gelling agent and the water are introduced, at the extruder inlet, at room temperature, and are then brought to the transportation zone at a temperature of about 50°C, after which they are blended and compressed in various zones of the extruder maintained at a temperature ranging from 60 to 100°C; the mass obtained is transported to the extruder outlet and extruded through a die.

27. Use according to either of Claims 25 and 26, **characterized in that** the rigid gel may be made anhydrous by dehydration of the aqueous gelled network by a standard drying process.

28. Use according to any one of Claims 25 to 27, **characterized in that** the water-soluble or hydrophilic gelling agent is chosen from the group formed by:
- alga extracts;
- seed extracts;
- plant exudates;
- microorganism exudates;
- fruit extracts;
- gelling agents of animal origin;
- water-soluble gelling synthetic polymers;
- silicon derivatives.

29. Use according to Claim 28, **characterized in that** the water-soluble gelling agent is chosen from the group formed by agar-agar, carageenans, alginates, carob gum, guar gum, gum arabic, karaya gum, gum tragacanth, ghatti gum, xanthan gum, cellulose or derivatives thereof, modified celluloses, pectins; gelatin, caseinates, crosslinked polyacrylic acids, synthetic hectorites, and aluminium and magnesium silicates.

30. Use according to any one of Claims 25 to 29, **characterized in that** the water-soluble or hydrophilic gelling agent is present in concentrations ranging from 20 to 80% by weight relative to the total weight of the composition.

31. Process for the preparation of the composition in rigid gel form as defined in any one of Claims 1 to 24, **characterized in that** it is obtained from at least one water-soluble or hydrophilic gelling agent, from the dermatological or cosmetic substance and optionally from the other constituents as defined in the preceding claims, in the presence of water by mixing, blending, compression and extrusion in a twin-screw extruder, after which the rigid gel is optionally dehydrated by a standard drying process.

32. Process according to Claim 31, **characterized in that** the starting materials are introduced, at the extruder inlet, at room temperature, and are then brought to the transportation zone at a temperature of about 50°C, after which they are blended and compressed in various zones of the extruder maintained at a temperature ranging from 60 to 100°C; the mass obtained is transported to the extruder outlet and extruded through a die.

33. Cosmetic treatment process for the care and/or conditioning and/or hygiene of the skin, the hair, the scalp or the mucous membranes, **characterized in that** an aqueous solid composition according to any one of Claims 1-2 and 4-21 is used, **in that** this composition is made moist at the surface with water and **in that** the said partially rehydrated composition is applied to the skin, the hair, the scalp or the mucous membranes.

34. Process for making up the lips, the face or the eyelids, **characterized in that** an aqueous solid composition according to any one of Claims 1-2 and 4-21 is used, **in that** this composition is made moist at the surface with water and **in that** the said partially rehydrated composition is applied to the lips, the face or the eyelids.
